# EUROPEAN PATENT SPECIFICATION

(11) **EP 0 439 504 B1**
(45) Date of publication and mention of the grant of the patent: **02.01.1997**
(21) Application number: 89911849.1
(22) Date of filing: 20.10.1989
(51) Int. Cl.: B65D 75/54, A61L 9/04

(54) **CONTAINER**
BEHÄLTER
RECIPIENT

(30) Priority: 22.10.1988 GB 8824799; 03.12.1988 GB 8828288
(43) Date of publication of application: 07.08.1991
(73) Proprietor: The Beautiful Bouquet Company Limited, St. James, Northampton NN5 5LH (GB)
(72) Inventor: Bishopp, Derek Albert, GB-Woodbridge Suffolk IP12 1LW (GB)
(74) Representative: Dummett, Thomas Ian Peter
(86) International application number: GB8901244
(87) International publication number: WO9004550

(56) References cited:
- EP-A- 0 283 621
- WO-A-87/03563
- BE-A- 692 754
- US-A- 3 216 882
- US-A- 4 874 129
- US-A- 4 880 690

## Description

The present invention relates to a container, notably to a scented container for rolls of toilet paper.

### BACKGROUND TO THE INVENTION:

Rolls of toilet paper are typically sold in packs containing two, four or more rolls so that the purchaser has to store the rolls which are not immediately required, usually in an unwrapped condition once the outer wrapping for the packs has been removed to gain access to the first roll. However, in the interests of economy, the packaging for such packs of toilet rolls is flimsy and often has no aesthetic appeal. The spare rolls are therefore stored out of sight and often not within immediate reach of the toilet where they are required. For example, they may be stored under a kitchen sink or in an airing cupboard. However, such locations are often under conditions which adversely affect the quality of the tissue paper from which the toilet paper is made.

The manufacturers of such paper impose strict quality controls on moisture and on chemicals in the paper so that it is acceptable to touch and does not bring potentially hazardous chemicals into contact with sensitive skin. However, when stored by the user much of the quality control is negated, for example when unwrapped toilet rolls are stored in the immediate vicinity of bleach or other strong chemical or abrasive cleaners. Where an unwrapped roll is stored adjacent a hot water cylinder, the paper loses up to 5% of its weight in 12 hours as the moisture required to retain its soft texture evaporates.

It has been proposed to impregnate toilet paper with a scent composition, but this requires a change to the conventional toilet paper manufacturing techniques. It has also been proposed to incorporate a scent sachet or block into a toilet paper pack to impart a scent to the paper, see for example US Patent Specifications Nos 3,711,024 and 4,513, 862. However, the scent composition was either in direct contact with the toilet paper or was located so that it would permeate preferentially into the interior of the paper container rather than into the environment, so as to impart scent to the paper as it was being used.

Scent compositions typically contain aromatic oils and anti-oxidants and these can be strongly absorbed by toilet paper if they are allowed to come into contact with the paper. As a result, such impregnated papers could give rise to allergic reactions and discomfort for a user of the paper.

Furthermore, by impregnating the toilet paper itself or by locating the scent carrier inside the packing for the paper, the scent is only available after the package has been opened. As a result, such packages can not be used both to retain the characteristics of the paper, such as moisture content, and the integrity of the package against intrusion from chemicals in the environment (which require that the package remain closed); and to permit escape of a scent into the environment during storage prior to use (which requires that the package be opened). As a result, there is no incentive for a user to store a spare roll in the toilet since it could not release its scent until actually in use.

In US Patent Specification No A-3,575,345 there is disclosed a sachet containing a pad of paper impregnated with a fluid perfume or deodorizing compound. When the end of the sachet is removed, the end of the paper pad is exposed and the pad can be pulled partially out of the sachet to cause the perfume or deodorizing compound to evaporate from the exposed pad. The rate of release of the perfume or deodorizing compound can be increased by pulling more of the pad out of the open end of the sachet. The sachet can be mounted in any desired location by means of adhesive carried on an outer wall of the sachet. Such a sachet requires the use of a paper or similar pad to carry the perfume or deodorising compound and once opened cannot be resealed. There is no reference to the use of such a sachet on a container for toilet paper which could be actuated when a user removes part or all of a wall of the container to remove material from within the container.

In British Patent No 430,577 there is described a toilet paper holder which incorporates a perforated holder for a scent block which allows scent to permeate both onto the paper during use and into the environment around the holder. However, such a holder does not form part of the packaging for the toilet paper and requires that the packaging around the paper be at least partially removed prior to insertion of the paper into the holder. As a result, not only is the paper exposed during use to the scent composition, with the attendant risk of contamination of the paper, but the scent holder does not form part of the packaging within which the toilet paper is sold and stored prior to use, thus requiring that the holder be purchased as a separate item. Again, there is no incentive for a user to store a spare packet of toilet paper in the toilet.

In PCT Published Application No W087/03563 is has been proposed to provide a strip of encapsulated scent upon the external face of a card carrying a blister pack housing a bottle of that scent. A prospective purchaser can sample the scent by rupturing the capsules to release the scent therein. However, the objective is to provide an external sample of what is to be found within the container. This is different from the objective in the present invention which is to provide an external source of scent, which is not the material within the container, so as to release scent into the environment of the container. The scent which is released has no direct relationship with the contents of the containers of the invention.

According to US-A-3 216 882 a porous plastic film is provided, wherein a plurality of pores extends throughout a dimension of the film. The film can be immersed into a volatile material to provide a volatile material releasing means.

The present invention relates to a form of scent release means for use on a container for a toilet roll which reduces the above conflicting problems with existing designs and enables a toilet roll to be stored prior to use under controlled conditions (thus preserving its quality and protecting it against contamination), which can be made visually attractive and releases a scent or other volatile material into its environment prior to use (thus providing an incentive to a user to store a spare toilet roll in the toilet) without the need for the user to purchase anything other than the toilet roll and its packaging.

### SUMMARY OF THE INVENTION:

The invention provides a flexible walled container (2) for the storage and transport of toilet paper (1) in sheet or roll form prior to use thereof, which container (2) is adapted to have at least part of a wall thereof removed prior to use of the toilet paper (1) so as to gain access to the toilet paper within the container (2), the container being provided with means (5, 6) for releasing a volatile material located on an outer wall (2) of the container whereby it is adapted to release the volatile material substantially only externally of the container into the environment of the container and substantially not into the interior of the container, characterised in that the means (5, 6) comprises:
a. a generally planar microporous plastic carrier member (5) secured to a wall of the container (2) which provides a vapour barrier between the volatile material and the wall of the container (2) and which is impregnated with a liquid composition containing a liquid volatile material which has been applied directly to a selected area of one face of said carrier member (5); and
b. a vapour impervious cover layer member (6) applied directly over at least the said selected area of the carrier member (5) and removably secured to said carrier member (5) so as to form with the carrier member (5) a substantially vapour impermeable enclosure (5, 6) for the liquid volatile material, whereby release of the volatile material from the carrier member (5) can be achieved by removing part or all of the said cover layer member (6) to expose the area of the carrier member (5) impregnated with the fluid composition directly to the environment of the releasing means (5, 6) so as to permit the volatile material to evaporate from the exposed surface of the carrier member (5).

Preferably, the volatile material releasing means is adapted to be actuated to release the volatile material prior to removal of the container wall to permit access to the contents of the container.

The container is made from a flexible material, notably a cardboard, card or paper or a sheet polymer; and in a preferred form comprises a paper, sheet plastic or similar wrapping around a roll of toilet paper; and the means for releasing the scent or other volatile material is carried on the exterior surface of the wrapping so that removal of part or all of the cover layer member releases volatile material from the exposed carrier member so that the releasing means can be actuated by a user when the container is exposed to a desired environment.

The term toilet paper is used herein in general terms to denote any unitary package containing sheets of toilet paper. The term thus includes generally square or rectangular packs of interleaved single sheets of toilet paper, facial tissues or other absorbent papers, as well as generally cylindrical rolls of toilet paper. For convenience, the invention will be described hereinafter in terms of a conventional perforated roll of toilet paper, notably a roll of absorbent tissue paper.

The container of the invention can take a wide range of forms, for example tubular containers or boxes adapted to hold a number of rolls or blocks of interleaved sheets. For example, the invention can be applied to a generally rectangular box-like container adapted to hold one, two or three rolls of toilet paper. Alternatively, the container can be provided as a paper or similar wrapper around the roll or rolls of toilet paper.

However, the invention is of especial use with toilet rolls each contained in an individual paper or plastics film wrapping. Such toilet rolls can be contained in an outer wrapping, for example a plastic outer bag from which the individual rolls are removed prior to placing in the toilet room; or each individual wrapping can carry a removable cover or secondary wrapping which is removed to activate the release of scent or other volatile material from the releasing means as described below.

It is preferred that the container totally encloses the toilet roll(s) and be generally closed so that the individual rolls are maintained in a controlled environment until unwrapped for use and so that the scent or other volatile material released from the releasing means carried by the container can not penetrate to any significant into the contents of the container. Thus, in a preferred form, the container takes the form of a paper or sheet plastic sleeve around the roll of paper having its open ends gathered together to form a substantially closed wrapping around the roll. The open ends are closed, for example by being stuffed into the open ends of the cardboard tube upon which such rolls are typically wound. A paper or similar disc held in place by a suitable adhesive is affixed to the wrapping across the open ends of the tube to retain the wrapping in position. Such a sleeve is typically formed by wrapping a sheet of paper around the roll so that the edges of the sheet overlap along an axial line along the roll. If desired, adhesive can be applied along part or all of the overlap to retain the wrapping in the form of a sleeve. Although such a form of wrapping is not completely airtight, such wrapping is acceptable for present purposes. Alternatively, a thermoplastic sheet material can be used as the wrapping material and the overlaps and joints heat sealed together to provide improved air-tightness to the container. If desired, such a thermoplastic material can be heat shrunk upon the toilet roll.

The container for the roll(s) of toilet paper is provided with a means for releasing a scent or other volatile material preferentially into the environment around the container so that little or none of the composition containing the scent or other volatile material or its carrier medium contacts the toilet paper within the container, notably before the container is opened to permit access to the contents for use.

The source of scent or other volatile material for use in the containers of the invention takes the form of a carrier member as defined in claim 1 to 6 impregnated with the desired scent or other liquid volatile material, which is affixed to an outer surface of a wall of the container, for example as a microporous plastic disc or other shaped panel, impregnated with an oil based scent composition.

The carrier member for the scent or other volatile material composition may also serve as the closure to the container and its removal permits access to the container for removal of the contents of the container whilst the scent or other volatile material is released during use of the contents. In this case it will be preferred that the volatile material is located some distance, typically 2 to 5 cms, from the aperture formed in the container wall so that little or none of the volatile material enters the container to contaminate the contents.

According to the invention means are provided by which the release of the scent or other volatile material can be delayed until the container is placed into the environment into which the scent or other volatile material is to be released. This is achieved by the provision of a vapour impervious cover layer member, for example, by applying a foil or similar tear off cover over the scent, which cover is removed to activate release of the scent.

Thus the invention provides a container where the liquid volatile material is impregnated into a generally planar microporous plastic carrier substrate which is applied to the exterior of a wall of the container, the substrate being provided with a closure member overlying the area impregnated with the liquid volatile material to provide a closure which is to be removed by a user in order to permit release of the volatile material from the substrate.

As stated above, the volatile material is released preferentially into the environment around the container and not significantly into the interior of the container. This is achieved as described above by providing the volatile material in a carrier disc or strip affixed to the exterior of the container. In order to minimise penetration of the liquid volatile material or its carrier medium through the wall of the container it may be desirable to provide a plastic sheet or other vapour barrier member between the carrier member and the container wall. Thus, where a scented disc is applied, this will carry the liquid scent on a vapour impervious carrier member, or the wall of the container can be coated with a suitable plastic film forming composition, eg. a wax or resin solution, over the desired area.

The container walls can be printed with suitable decorative motifs or panels so that the container presents an attractive external appearance to the observer.

The container can be manufactured using conventional techniques. If desired, the container can incorporate other features to enhance its utility. Thus, the cover layer which is to be removed to release the scent can be formed with apertures having foldable flaps or louvres, which can be opened or closed to provide a measure of control of the release of the scent into the surroundings.

The container of the invention provides a means by which a toilet roll can be stored in a controlled environment prior to use, thus retaining the quality intended by the manufacturer, in a visually attractive and distinctive form which will promote the manufacturer's image and which will provide a source of scent to combat odours at the same time, thus encouraging the user to have a spare toilet roll accessible in the toilet.

If desired, one or more containers of the invention can be incorporated into a conventional multiple pack of toilet rolls so that a purchaser is provided with one or more prepacked containers suitable for storage for future use, together with one or more toilet rolls ready for immediate use.

As indicated above, the containers of the invention can be made using conventional techniques and materials so that the invention can readily be achieved without significant disruption of existing toilet roll manufacturing and packing lines. This is especially the case where the container is wrapped in a conventional paper wrapper to which a scented disc is subsequently applied.

The invention has been described above in terms of a container for a roll of toilet paper. However, it will be appreciated that the paper need not be intended for use on the toilet, but could be any tissue paper for use upon the person. Thus, the invention can be applied to boxes of face tissues or paper handkerchiefs. Furthermore, the volatile material to be released into the environment of the container need not be merely to combat odours. Thus, the material may contain a medicament, eg. menthol or eucalyptus oil, and the term scent as used herein is to denote any liquid volatile material which it is desired to release into the environment of the container.

### DESCRIPTION OF THE DRAWINGS:

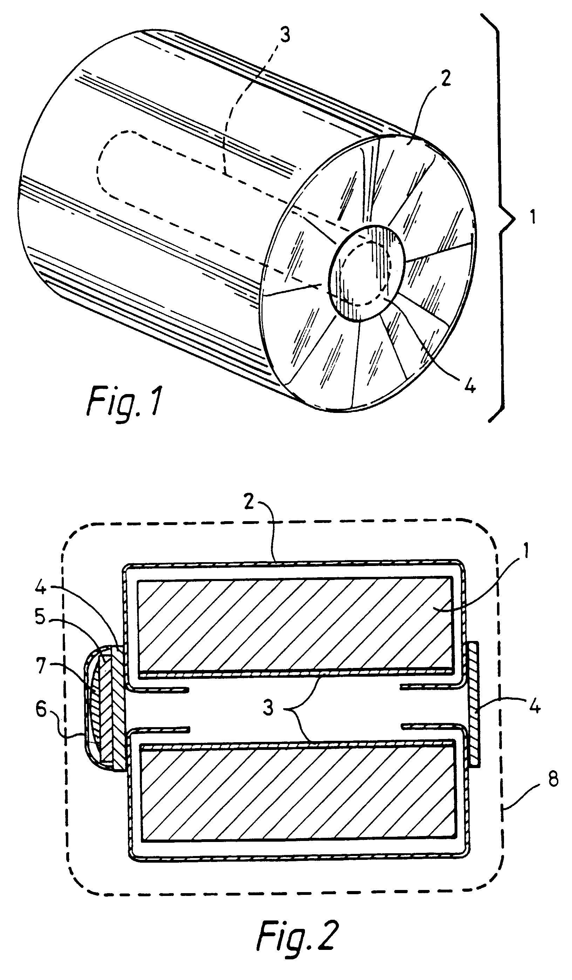

The invention will be described by way of illustration with respect to the accompanying drawings in which Figure 1 is a perspective view of a toilet roll; and Figure 2 is a vertical sectional view through the toilet roll of Figure 1.

### DESCRIPTION OF THE PREFERRED EMBODIMENT:

The toilet roll package of Figure 1 comprises a conventional roll of perforated tissue paper 1 in a substantially airtight wrapping of a paper sleeve 2 having each of its ends gathered together into the tube 3 upon which the paper roll is wound. The ends of the sleeve are sealed with a paper disc 4 held in place by a PVA or other suitable adhesive. Such a wrapped toilet roll can be produced from conventional materials and by conventional techniques.

The toilet roll is provided with a scent disc 5 which can either be formed integrally with disc 4 or can be affixed as a separate component to the package. As shown, the disc 5 has a peel off outer plastic, paper or metal foil cover layer 6 which exposes the scent when it is desired to activate the release of scent into the environment around the package. The drawing shows a conventional form of a scent disc in which the liquid scent is carried by a pad 7. However, according to the present invention the pad 7 is dispensed with and the liquid scent impregnated directly into the disc 5. In this way the generally airtight packaging around the toilet paper itself is retained whilst the scent disc is exposed to the environment.

When the toilet paper is required, the paper wrapping 2 is removed and discarded, thus exposing the toilet paper for access by a user.

The package of Figure 1 can also or alternatively be wrapped in a removable outer paper or similar wrapping 8 which is removed to expose the package and its scent disc 5 when it is desired to place the package in the toilet for imminent use and to expose the disc 5 to the environment.

The outer face of the sleeve 2 can be printed with decorative motifs or the like to make the package more aesthetically attractive and can also carry the name of the manufacturer so that a user is reminded of the name of the manufacturer at all times. The invention can be applied to sample boxes or sachets of facial tissues with the scent disc containing a particular perfume which a perfume manufacturer wishes to promote.

The scented discs for use in the invention are novel and the invention provides a generally planar volatile material releasing means adapted to be secured to a substrate and selectively to release a volatile material to the environment of the means by volatilization of the volatile material from an exposed surface of the planar member, characterised in that it comprises:
a. a generally planar microporous plastic carrier member (5) being adapted to provide a vapour barrier between the volatile material and a substrate to which the means is secured and being impregnated with a liquid composition containing a liquid volatile material which has been applied directly to a selected area of one face of said carrier member (5); and
b. a vapour impervious cover layer member (6) applied directly over at least the said selected area of the carrier member (5) and removably secured to said carrier member (5) so as to form with the carrier member (5) a substantially vapour impermeable enclosure (5, 6) for the liquid volatile material, whereby release of the volatile material from the carrier member (5) can be achieved by removing part or all of the said cover layer member (6) to expose the area of the carrier member (5) impregnated with the fluid composition directly to the environment of the releasing means (5, 6) so as to permit the volatile material to evaporate from the exposed surface of the carrier member (5).

It has been proposed, in for example US Patents Nos A-3,575,345, A-4,874,129, A-4,880,690 and A-4,957,246, to incorporate a scent into a polymer monomer or oligomer which is then cured to incorporate the scent into the solid polymer. However, such proposals require the use of catalysts and other chemicals and actinic radiation and/or heating to cure the monomer or oligomer and this causes degradation of the scent and/or loss of the more volatile fractions of the scent. As a result such proposals do not enable a true rendition of the scent to be achieved. In the releasing means of the invention, the volatile material is a liquid and is present in a liquid composition, for example as a fragrance oil or medicament oil, impregnated into the base layer. The need to form polymer solids and such heating, radiation or chemical reaction is avoided in the production of the volatile material releasing means of the invention is thus avoided.

Preferably, the other face of the carrier member is provided with an adhesive layer whereby the releasing means can be affixed to a substrate. Preferably, the adhesive is a pressure sensitive adhesive applied as a coating to one face of the carrier member and the volatile material composition is a liquid scent composition applied as a layer to the other face of the carrier member; and both the adhesive and scent are covered by removable cover layers, eg. of paper, plastics or metal foil.

## Claims

1. A generally planar volatile material releasing means adapted to be secured to a substrate and selectively to release a volatile material to the environment of the means by volatilization of the volatile material from an exposed surface of the planar member, characterised in that it comprises:
a. a generally planar microporous plastic carrier member (5) being adapted to provide a vapour barrier between the volatile material and a substrate to which the means is secured and being impregnated with a liquid composition containing a liquid volatile material which has been applied directly to a selected area of one face of said carrier member (5); and
b. a vapour impervious cover layer member (6) applied directly over at least the said selected area of the carrier member (5) and removably secured to said carrier member (5) so as to form with the carrier member (5) a substantially vapour impermeable enclosure (5, 6) for the liquid volatile material, whereby release of the volatile material from the carrier member (5) can be achieved by removing part or all of the said cover layer member (6) to expose the area of the carrier member (5) impregnated with the fluid composition directly to the environment of the releasing means (5, 6) so as to permit the volatile material to evaporate from the exposed surface of the carrier member (5).

2. A means as claimed in claim 1, characterised in that the carrier member (5) has applied to the reverse side to that impregnated with the liquid composition an adhesive whereby the carrier member (5) may be affixed to the substrate.

3. A means as claimed in either of claims 1 or 2, characterised in that the liquid composition comprises a liquid fragrance or medicament oil.

4. A means as claimed in any one of claims 1 to 3, characterised in that the means is applied to an external wall (2) of a flexible walled storage and transport container for a material, whereby the means (5, 6) is adapted to release the said liquid volatile material externally of the container upon removal of said cover layer member (6) to expose said carrier member (5).

5. A means as claimed in claim 4, characterised in that said cover layer member (6) forms at least part of a closure (5, 6) to an aperture in a wall of the container (2) through which aperture the material (1) contained in the container (2) is to be removed.

6. A flexible walled container (2) for the storage and transport of toilet paper (1) in sheet or roll form prior to use thereof, which container (2) is adapted to have at least part of a wall thereof removed prior to use of the toilet paper (1) so as to gain access to the toilet paper within the container (2), the container being provided with means (5, 6) for releasing a volatile material located on an outer wall (2) of the container whereby it is adapted to release the volatile material substantially only externally of the container into the environment of the container and substantially not into the interior of the container, characterised in that the means (5, 6) comprises:
a. a generally planar microporous plastic carrier member (5) secured to a wall of the container (2) which provides a vapour barrier between the volatile material and the wall of the container (2) and which is impregnated with a liquid composition containing a liquid volatile material which has been applied directly to a selected area of one face of said carrier member (5); and
b. a vapour impervious cover layer member (6) applied directly over at least the said selected area of the carrier member (5) and removably secured to said carrier member (5) so as to form with the carrier member (5) a substantially vapour impermeable enclosure (5, 6) for the liquid volatile material, whereby release of the volatile material from the carrier member (5) can be achieved by removing part or all of the said cover layer member (6) to expose the area of the carrier member (5) impregnated with the fluid composition directly to the environment of the releasing means (5, 6) so as to permit the volatile material to evaporate from the exposed surface of the carrier member (5).

7. A container (2) as claimed in claim 6, characterised in that removal of at least part of the container wall (2) to gain access to the contents of the container is adapted to cause release of the volatile material.

8. A container (2) as claimed in either of claims 6 or 7, characterised in that the container comprises a paper or similar wrapping (2) around a roll of toilet paper (1) and the means for releasing the volatile material (5, 6) is carried on the exterior surface of the wrapping (2) whereby release of the volatile material from the means (5, 6) can be actuated by a user when the container (2) is exposed to a desired environment.

9. A container (2) as claimed in claim 8, characterised in that the container (2) comprises a generally tubular wrapping (2) around a generally cylindrical roll of toilet paper (1), the open ends of the wrapping being gathered together at each end of the roll to form a closure to the container and the carrier member (5) is applied to at least one end of the resulting container.

10. A container (2) as claimed in any one of claims 7 to 9, characterised in that the liquid composition contains a liquid fragrance or medicament oil.

## Patentansprüche

1. Im wesentlichen ebenes, flüchtiges Material freisetzendes Mittel, welches ausgebildet ist zur Anbringung auf einem Träger und wählbar ein flüchtiges Material in die Umgebung des Mittels freizusetzen, durch Verdunstung des flüchtigen Materials von einer offenen Oberfläche des ebenen Teils,
dadurch gekennzeichnet,
daß es umfaßt:
a. ein im wesentlichen ebenes, mikroporöses, aus Kunststoff bestehendes Trägerteil (5), welches zum Vorsehen einer Dampfsperre zwischen dem flüchtigen Material und einem Träger ausgebildet ist, mit dem das Mittel verbunden ist, und welches mit einer flüssigen Zusammensetzung imprägniert ist, welche ein flüssiges, flüchtiges Material enthält, welches direkt auf einen bestimmten Bereich einer Oberfläche des genannten Trägerteils (5) aufgebracht worden ist; und
b. ein für Dampf undurchlässiges Abdeckschichtteil (6), welches direkt über den bestimmten Bereich des Trägerteils (5) angebracht ist und entfernbar mit dem Trägerteil (5) so verbunden ist, daß es mit dem Trägerteil (5) einen im wesentlichen für Dampf undurchlässigen Abschluß (5, 6) für das flüssige, flüchtige Material bildet, wobei das Freisetzen des flüchtigen Materials aus dem Trägerteil (5) dadurch erreicht werden kann, daß man einen Teilbereich des genannten Abdeckschichtteils (6) oder dieses insgesamt entfernt, um den mit der flüssigen Zusammensetzung imprägnierten Bereich des Trägerteils (5) direkt der Umgebung des freisetzenden Mittels (5, 6) auszusetzen, um dem flüchtigen Material ein Abdampfen von der offenen Oberfläche des Trägerteils (5) zu erlauben.

2. Mittel nach Anspruch 1,
dadurch gekennzeichnet,
daß das Trägerteil (5) auf seiner Seite, die der mit der flüssigen Zusammensetzung imprägnierten Seite gegenüberliegt, einen Kleber aufweist, durch den das Trägerteil am Träger befestigt werden kann.

3. Mittel nach einem der Ansprüche 1 oder 2,
dadurch gekennzeichnet,
daß die flüssige Zusammensetzung einen flüssigen Duftstoff oder ein medizinisches Öl umfaßt.

4. Mittel nach einem der Ansprüche 1 bis 3,
dadurch gekennzeichnet,
daß das Mittel an der äußeren Wand (2) eines mit flexiblen Wänden versehenen Lager- und Transportbehälters für ein Material angebracht ist, wodurch das Mittel (5, 6) dazu ausgebildet ist, das genannte flüssige, flüchtige Material bei Entfernen des Abdeckschichtteils (6) in das Behälteräußere freizusetzen und das Trägerteil freizulegen.

5. Mittel nach Anspruch 4,
dadurch gekennzeichnet,
daß das Abdeckschichtteil (6) mindestens einen Teil eines Abschlusses (5, 6) gegenüber einer Öffnung in einer Wand des Behälters (2) bildet, durch welche Öffnung das im Behälter (2) enthaltene Material (1) entfernt werden kann.

6. Mit flexiblen Wänden versehener Behälter (2) für das Aufbewahren und den Transport von Toilettenpapier (1) in Blatt- oder Rollenform vor dessen Benutzung, welcher Behälter (2) so ausgebildet ist, daß zumindest ein Teil seiner Wand vor Benutzung des Toilettenpapiers (1) entfernt werden kann, damit man Zugang zu dem Toilettenpapier innerhalb des Behälters (2) erhält, wobei der Behälter mit an einer äußeren Wand des Behälters (2) angeordneten Mitteln (5, 6) für ein Freisetzen eines flüchtigen Materials versehen ist, welche an einer äußeren Wand (2) des Behälters angeordnet sind, wodurch er ausgebildet ist, das flüchtige Material lediglich nach außen aus dem Behälter in die Umgebung des Behälters abzugeben und im wesentlichen nicht in das Behälterinnere, dadurch gekennzeichnet,
daß das Mittel (5, 6) umfaßt:
a. ein im wesentlichen ebenes, mikroporöses, aus Kunststoff bestehendes Trägerteil (5), welches an einer Wand des Behälters befestigt ist und welches eine Dampfsperre zwischen dem flüchtigen Material und der Wand des Behälters bildet und welches mit einer flüssigen Zusammensetzung imprägniert ist, welche ein flüssiges, flüchtiges Material enthält, das direkt auf einen bestimmten Bereich einer Oberfläche des genannten Trägerteils (5) aufgebracht worden ist; und
b. ein für Dampf undurchlässiges Abdeckschichtteil (6), welches direkt über mindestens den genannten bestimmten Bereich des Trägerteils (5) angebracht ist und entfernbar mit dem Trägerteil (5) so verbunden ist, daß es mit dem Trägerteil (5) einen im wesentlichen für Dampf undurchlässigen Abschluß (5, 6) für das flüssige, flüchtige Material bildet, wodurch das Freisetzen des flüchtigen Materials aus dem Trägerteil (5) dadurch erreicht werden kann, daß man einen Teilbereich des genannten Abdeckschichtteils (6) oder dieses insgesamt entfernt, um den mit der flüssigen Zusammensetzung imprägnierten Bereich des Trägerteils (5) direkt der Umgebung des freisetzenden Mittels (5, 6) freizuetzen, um dem flüchtigen Material ein Abdampfen von der offenen Oberfläche des Trägerteils (5) zu erlauben.

7. Behälter (2) nach Anspruch 6,
dadurch gekennzeichnet,
daß das Entfernen von mindestens einem Teil der Behälterwand (2) zum Erhalten eines Zugangs zum Inneren des Behälters ausgebildet ist, um ein Freisetzen des flüchtigen Materials zu veranlassen.

8. Behälter (2) nach einem der Ansprüche 6 oder 7,
dadurch gekennzeichnet,
daß der Behälter eine aus Papier oder ähnlichem Material bestehende Umhüllung (2) um eine Rolle von Toilettenpapier (1) umfaßt und das Mittel zum Freisetzen des flüchtigen Materials (5, 6) auf der äußeren Oberfläche der Umhüllung (2) gehalten wird, wodurch das Freisetzen des flüchtigen Materials aus dem Mittel (5, 6) vom Benutzer bewirkt werden kann, wenn der Behälter (2) einer gewünschten Umgebung ausgesetzt ist.

9. Behälter (2) nach Anspruch 8,
dadurch gekennzeichnet,
daß der Behälter (2) eine im wesentlichen röhrenförmige Umhüllung (2) um eine im wesentlichen zylindrische Rolle von Toilettenpapier (1) umfaßt, wobei die offenen Enden der Umhüllung an jedem Ende der Rolle zusammengefügt sind, um einen Verschluß für den Behälter zu bilden und daß das Trägerteil (5) an mindestens einem Ende des gebildeten Behälters angebracht ist.

10. Behälter (2) nach einem der Ansprüche 7 bis 9,
dadurch gekennzeichnet,
daß die flüssige Zusammensetzung einen flüssigen Duftstoff oder medizinisches Öl enthält.

## Revendications

1. Dispositif plan dans l'ensemble libérant une substance volatile, adapté pour être fixé à un substrat et pour libérer de manière sélective une substance volatile dans l'environnement du dispositif par vaporisation de la substance volatile à partir d'une surface exposée de l'élément plan, caractérisé en ce qu'il comprend:
a. un élément de support (5) en plastique microporeux, plan dans l'ensemble, étant adapté pour fournir un écran pare-vapeur entre la substance volatile et un substrat auquel le dispositif est fixé et étant imprégné d'une composition liquide contenant une substance liquide volatile qui a été appliquée directement sur une zone choisie d'une face dudit élément de support (5); et
b. un élément de couvercle (6) étanche à la vapeur appliqué directement au-dessus d'au moins ladite zone choisie de l'élément de support (5) et fixé de façon amovible audit élément de support (5) de manière à former avec l'élément de support (5) une enceinte (5, 6) sensiblement imperméable à la vapeur pour la substance liquide volatile, la libération de la substance volatile à partir de l'élément de support (5) pouvant être effectuée en retirant une partie ou la totalité dudit élément de couvercle (6) afin d'exposer la zone de l'élément de support (5) imprégnée avec la composition liquide directement à l'environnement du dispositif de libération (5, 6) de façon à permettre à la substance volatile de s'évaporer de la surface exposée de l'élément de support (5).

2. Dispositif selon la revendication 1, caractérisé en ce que l'élément de support (5) porte un adhésif appliqué sur la face opposée à celle imprégnée avec la composition liquide, l'élément de support (5) pouvant être fixé au substrat.

3. Dispositif selon l'une des revendications 1 ou 2, caractérisé en ce que la composition liquide comprend un parfum liquide ou une huile médicamenteuse.

4. Dispositif selon l'une quelconque des revendications 1 à 3, caractérisé en ce que le dispositif est appliqué contre une paroi extérieure (2) d'un récipient à parois flexibles de stockage et de transport d'un matériau, le dispositif étant adapté pour délivrer ladite substance liquide volatile à l'extérieur du récipient par retrait dudit élément de couvercle (6) pour exposer ledit élément de support (5).

5. Dispositif selon la revendication 4, caractérisé en ce que ledit élément de couvercle (6) forme au moins une partie d'une fermeture (5, 6) pour une ouverture dans une paroi du récipient (2) ouverture par laquelle le matériau (1) contenu dans le récipient (2) doit être retiré.

6. Récipient (2) à parois flexibles pour le stockage et le transport de papier toilette (1) sous forme de feuilles ou de rouleau avant l'utilisation de celui-ci, lequel récipient (2) est adapté pour qu'au moins une partie d'une paroi de celui-ci soit retirée avant d'utiliser le papier toilette (1) de manière à pouvoir accéder au papier toilette à l'intérieur du récipient (2), le récipient étant équipé d'un dispositif (5, 6) pour libérer une substance volatile située sur une paroi extérieure (2) du récipient, celui-ci étant adapté pour libérer la substance volatile sensiblement uniquement à l'extérieur du récipient dans l'environnement du récipient et sensiblement pas vers l'intérieur du récipient, caractérisé en ce que le dispositif comprend :
a. un élément de support (5) en plastique microporeux, plan dans l'ensemble, fixé à une paroi du récipient (2) qui fournit un écran pare-vapeur entre la substance volatile et la paroi du récipient (2) et qui est imprégné d'une composition liquide contenant une substance liquide volatile qui a été appliquée directement sur une zone choisie de l'une des faces dudit élément de support (5); et
b. un élément de couvercle (6) étanche à la vapeur appliqué directement au-dessus d'au moins ladite zone choisie de l'élément de support (5) et fixé de façon amovible audit élément de support (5) de manière à former avec l'élément de support (5) une enceinte (5, 6) sensiblement imperméable à la vapeur pour la substance liquide volatile, la libération de la substance volatile à partir de l'élément de support (5) pouvant être effectuée en retirant une partie ou la totalité dudit élément de couvercle (6) afin d'exposer la zone de l'élément de support (5) imprégnée avec la composition liquide directement à l'environnement du dispositif de libération (5, 6) de façon à permettre à la substance volatile de s'évaporer de la surface exposée de l'élément de support (5).

7. Récipient (2) selon la revendication 6, caractérisé en ce que le retrait d'au moins une partie de la paroi du récipient (2) afin de pouvoir accéder au contenu du récipient est prévu pour provoquer la libération de la substance volatile.

8. Récipient (2) selon l'une des revendications 6 ou 7, caractérisé en ce que le récipient comprend un papier ou autre emballage (2) autour d'un rouleau de papier toilette (1) et le dispositif pour libérer la substance volatile (5, 6) est supporté par la surface externe de l'emballage (2), la libération de la substance volatile à partir du dispositif (5, 6) pouvant être actionnée par un utilisateur lorsque le récipient (2) est exposé à un environnement voulu.

9. Récipient (2) selon la revendication 8, caractérisé en ce que le récipient (2) comprend un emballage (2) tubulaire dans l'ensemble, autour d'un rouleau de papier toilette (1) cylindrique dans l'ensemble, les extrémités ouvertes de l'emballage étant assemblées avec chaque extrémité du rouleau afin de former une fermeture pour le récipient et l'élément de support (5) est appliqué sur au moins une extrémité du récipient formé.

10. Récipient (2) selon l'une quelconque des revendications 7 à 9, caractérisé en ce que la composition liquide contient un parfum liquide ou une huile médicamenteuse.
